# EUROPEAN PATENT APPLICATION

(11) **EP 3 878 442 A1**
(43) Date of publication of application: **15.09.2021**
(21) Application number: 21156344.0
(22) Date of filing: 23.08.2013
(51) Int. Cl.: A61K 31/135, A61P 25/24, A61K 9/00, A61K 9/08, A61K 47/02, A61P 25/22, A61P 43/00

(54) **ANXIOLYTIC COMPOSITION COMPRISING KETAMINE, FORMULATION AND METHOD OF USE**

(30) Priority: 23.08.2012 US 201261692380 P
(62) Divisional of application: 13831683.1
(71) Applicant: Weg, Stuart L., Franklin Lakes, NJ 07417 (US)
(72) Inventor: Weg, Stuart L., Franklin Lakes, NJ 07417 (US)
(74) Representative: HGF

(57) **Abstract**

A method for treating, ameliorating or preventing the onset of anxiety in a subject comprises administering to such subject an NMDA receptor antagonist in an amount that is sub- anesthetic and hypo-analgetic. The NMDA receptor antagonist may comprise ketamine and its pharmaceutically acceptable salts, and is administered as a premedication. Instances of use in this manner include administration prior to an anxiety causing event, such as a medical or a dental procedure. The administration of the NMDA receptor antagonist composition is particularly useful as a premedication for adults

## Description

### BACKGROUND OF THE INVENTION

### Field of the Invention

The present invention relates to the area of pharmaceutical chemistry, and more particularly, to formulations and compositions for use in the prevention or treatment of anxiety and/or mild depression.

### Description of the Related Art

The present invention is primarily concerned with the treatment of adults who suffer from or exhibit anxiety. The following discussion generally reviews the condition of anxiety as it is understood in clinical (psychiatric) terms, however, and as stated later on herein, the present invention focuses on a less severe form of the condition, that is commonly experienced by virtually all individuals at some time in their lives. The following discussion should therefore be considered as a general exposition of the condition to identify the general state of the art.

Accordingly, "Anxiety" refers to an emotional state of apprehension or other unease that is distressing or otherwise unpleasant to a person. It is the central feature of various anxiety disorders, including, for example, generalized anxiety disorder, obsessive-compulsive disorder, panic disorder, phobic disorders, and stress disorders. Anxiety may also occur comorbidly with other mental disorders, such as with mixed-anxiety depression, or may be a symptom of them, such as in premenstrual dysphoric syndrome. Anxiety may also occur comorbidly with conditions other than mental disorders, such as with Alzheimer's disease or fibromyalgia, for example.

General anxiety disorder is characterized by excessive anxiety, often with little provocation. The anxiety often has more than one object (for example, finances and health) and changes over time. It is often accompanied by one or more physical symptoms, such as fatigue, headaches, muscle tension, muscle aches, difficulty swallowing, trembling, twitching, irritability, sweating, hot flashes, restlessness, and difficulty concentrating.

Obsessive-compulsive disorder is characterized by intrusive ideas (such as a fear of contamination, fear of flying) or impulses (such as inflicting harm on others) or a compulsion to perform certain behaviors in order to lessen the anxiety provoked by such ideas of impulses. The compulsions often involve repetitive behavior, such as repeatedly washing hands, counting, or uttering a certain phrase, and may or may not be observable to others.

A panic attack is characterized by an intense, often spontaneous episode of anxiety accompanied by one or more cognitive or somatic symptoms. Cognitive symptoms include a fear of dying, fear of going crazy or losing control, feelings of unreality, strangeness, or detachment from the environment. Somatic symptoms include chest pain or discomfort, dizziness, faintness, feeling of choking, flushes or chills, nausea or abdominal distress, numbness or tingling sensations, palpitations or accelerated heart rate, sensations of shortness of breath or smothering, sweating, and trembling or shaking. Panic attacks may occur spontaneously, or may occur in connection with other anxiety disorders; a person with claustrophobia, for example, may experience a panic attack when entering an elevator. Panic disorder occurs when a person repeatedly suffers panic attacks.

### Agoraphobia Without History of Panic Disorder

Agoraphobia is a condition characterized by the feature of anxiety about being in places or situations from which escape might be difficult (or embarrassing) or in which help may not be available in the event of having a panic attack or panic-like symptoms (e.g., fear of having a sudden attack of dizziness or a sudden attack of diarrhea). Agoraphobia occurs in the context of panic disorder with agoraphobia and agoraphobia without history of panic disorder. The essential features of agoraphobia without history of panic disorder are similar to those of panic disorder with agoraphobia except the focus of fear is on the occurrence of incapacitating or extremely embarrassing panic-like symptoms or limited symptom attacks rather than full panic attacks.

Almost all individuals (over 95%) who present with agoraphobia also have a current diagnosis (or history) or panic disorder. In contrast, the prevalence of agoraphobia without history of panic disorder in epidemiological samples has been reported to be higher than that for panic disorder with agoraphobia.

### Obsessive-Compulsive Disorder (OCD)

The primary symptom is recurrent obsessions (i.e., recurrent and intrusive thoughts, images or urges that cause marked anxiety) and/or compulsions (i.e., repetitive behaviors or mental acts that are performed to reduce the anxiety generated by one's obsessions) of sufficient severity to cause distress, be time consuming or to interfere significantly with a person's normal routine or lifestyle. Anxiety is an associated feature of this disorder: an affected person may, for example, show a phobic avoidance of situations that involve the cause of the obsession. Typical obsessions concern contamination, doubting (including self-doubt) and disturbing sexual or religious thoughts. Typical compulsions include washing, checking, ordering things, and counting.

### Social Phobia

Social phobia is characterized by the persistent fear of social or performance situations in which embarrassment may occur. Typical situations feared or avoided by individuals with social phoebe include parties, meetings, eating in front of others, writing in front of others, public speaking, conversations, meeting new people, and other related situations. Exposure to social or performance situations almost invariably provokes an immediate anxiety response, as well as sweating, trembling, racing or pounding heart beat, mental confusion, and a desire to flee. Social avoidance and isolation can also become extreme, especially in the more generalized condition. Alcohol abuse is more commonly associated with social phobia than any other anxiety disorder, and frequently represents an attempt at self medication of social fears.

### Post-Traumatic Stress Disorder (PTSD)

The principal characteristic symptoms involve re-experiencing a traumatic (i.e. psychologically distressing) event, the avoidance of stimuli associated with that event, the numbing of general responsiveness, and increased arousal. The "events" concerned are outside the range of common experiences such as simple bereavement, chronic illness and marital conflict.

### Generalized Anxiety Disorder (GAD)

GAD is a condition of which the essential feature is unrealistic or excessive anxiety, and worry about two or more life circumstances for six months or longer. The worry must be experienced as difficult to control and during that time the affected person is bothered by the concerns for more days than not. When the person is anxious he or she manifests signs of motor tension, autonomic hyperactivity and vigilance and scanning.

### Specific Phobia

Specific phobia is an anxiety disorder of which the essential feature is a persistent fear of a circumscribed stimulus, which may be an object or situation, other than fear of having a panic attack or of humiliation or embarrassment in social situations (which falls under social phobia). Examples include phobias of flying, heights, animals, injections, and blood. Simple phobias may be referred to as "specific" phobias and, in the population at large. Exposure to the phobic stimulus will almost invariably lead to an immediate anxiety response.

Multiple causes are suspected for anxiety disorders, especially a combination of genetic makeup, early growth and development, and later life experience. The anxiety disorders are treated with some form of counseling or psychotherapy or pharmacotherapy (drug therapy), either singly or in combination. The medications typically used to treat patients with anxiety disorders are benzodiazepines, selective serotonin reuptake inhibitors (SSRIs), and buspirone.

Narcotics that function as analgesics, such as cocaine have been used in the past for conditions associated with depression. Morphine elixers were used extensively for depression as were cocaine formulations for the treatment of depression and associated anxiety. While there are numerous instances of drugs such as opioids other narcotic agents and the like being used for the treatment of depression and /or anxiety, all of the foregoing suffer from the drawback that they are habit-forming and strongly induce dependence among the population to which they are administered. As a result, it is desirable to minimize and strongly control the use and administration of such agents

The benzodiazepines are a large class of relatively safe and widely prescribed medications that have rapid and profound antianxiety and sedative-hypnotic effects. Drugs within the SSRI class are used for the treatment of anxiety disorders such as panic disorder, agoraphobia, OCD, social phobia, post-traumatic stress disorder, specific phobia and broader anxiety disorders [Kaplan & Sadock's Comprehensive textbook of psychiatry 7th. edition, 1, 1441-1498 (1999)]. Buspirone is a relatively selective 5HT_{1A} partial agonist, approved by the FDA as an anxiolytic, most useful for the treatment of GAD, and now frequently used as an adjunct to SSRIs [Kaplan & Sadock's Comprehensive textbook of psychiatry 7th. edition, 1, 1441-1498 (1999)].

There appears to be effective pharmacological and psychological treatments for GAD. Although almost medication studies are based on old criteria for GAD (which have since been substantially revised), there is evidence that a range of pharmacological interventions may be helpful for GAD, including buspirone, imipramine and a variety of benzodiazepines. Pharmacotherapy is considered less effective in GAD than in some other anxiety disorders (Kaplan & Sadock's Comprehensive textbook of psychiatry 7th. edition, 1, 1441-1498 (1999)]. Treatment is usually behavioral exposure. Medications are used occasionally to alleviate the anticipatory anxiety associated with beginning exposure treatment. Low-dose benzodiazepines and β-adrenergic receptor antagonists can be used for this purpose on an as-needed basis.

Concerns have been expressed over possible side effect of some of the medications used to treat anxiety disorders, particularly the benzodiazepines. Common side effects associated with these medications, which may decrease over the course of treatment, include sedation, fatigue, ataxia, slurred speech, and amnesia, the latter two effects observable if the agents are used in high doses, or are otherwise abused. Benzodiadepines have also the potential for producing drug dependence (i.e. physiological or behavioral symptoms after discontinuation of use), and in this context, suffer from the same drawbacks recited with respect to the narcotic agents discussed.

There is therefore a continuing need for new agents that are effective and safe anxiolytics. More specifically, a need exists for the development of a dosage form and composition for the administration of a medication that can function as an anxiolytic, and that leaves no anesthetic effect, and that does not induce addiction or dependence or potential for respiratory or circulatory collapse/impairment. Also, it is toward the achievement of the aforementioned objectives that the present invention is directed.

### SUMMARY OF INVENTION

In accordance with the present invention, a method for inhibiting or treating the development of anxiety in a subject is disclosed which comprises administering to the subject an anxiolytic agent comprising an NMDA receptor antagonist, in an amount which is non-anesthetic and mildly analgesic. More particularly, the NMDA receptor antagonist may be prepared in a composition in an exemplary unit dosage amount of between about 0.05mg/kg to 0.5mg/kg, with 0.1mg/kg to about 0.3mg/kg, being exemplary.

The present method comprises the administration of the anxiolytic agent by a variety of routes, including intravenous, intranasal, transdermal, transmucosal and transbuccal administration.

The invention relates to the use of NMDA receptor antagonists, and preparations containing the same, and the manufacture of pharmaceutical compositions, all of which can be used to relieve natural occurrences of anxiety as well as symptoms of anxiety disorders.

More particularly, the present invention is concerned with anxiety that is of a less severe nature, and could be best described as the feeling of uneasiness and apprehension that is frequently experienced by normal individuals. This form of anxiety is distinguishable from the clinical or psychiatric state that has been discussed in detail above. While the disorders described here can interfere with a person's level of functioning, but can be effectively treated by use of the present invention. The compounds of the invention are particularly useful for treating anxiety that is not severe. A person with occasional panic attacks, for example, may be treated with compounds of the invention even though the person may not have panic disorder; the person need not wait to suffer from repeated panic attacks or be incapacitated by them before starting treatment with the compounds of the present invention. Similarly, a patient suffering from a mild form of acute stress disorder may be treated with compounds of the invention; one need not wait for the acute disorder to progress to posttraumatic stress disorder. What matters is only that a person seeking treatment for anxiety finds the anxiety unpleasant and wishes to alleviate it and/or prevent it from occurring.

The composition, formulation and device of the invention is likewise embodied in a kit, where, for example, a suitable unit dosage form may be prepared in a single-use aerosol or multi dose spray device for personal storage and immediate use.

Accordingly, it is a principal object of the present invention to prepare a formulation and composition for the administration of an anxiolytic that prevents, ameliorates or treats anxiety in a subject without causing cognitive impairment and little or no negative impact on patient function/performance.

It is accordingly, a further object of the present invention to provide a formulation, composition and method of administration as aforesaid, that offers rapid onset and relief with no negative after effects.

It is a yet further object of the present invention to provide a unit dosage form and corresponding kit including the formulation and composition of the present invention disposed within separable containers within an administration device such as a syringe.

Other objects and advantages will become apparent to those skilled in the art from a review of the ensuing detailed description.

### DETAILED DESCRIPTION OF THE INVENTION

In accordance with the present invention, the foregoing objects and advantages are readily attained.

In its broadest aspect, the present invention relates to a composition and a formulation that unexpectedly functions as a safe and effective anxiolytic. The present invention comprises the administration of an NMDA receptor antagonist, such as ketamine at a sub-anesthetic dose, for use as an anxiolytic that is particularly suited to treat the mild form of anxiety of interest herein. The method, composition and dosage form are specifically intended for use with adult subjects, from adolescent age and older.

NMDA receptor antagonists are therapeutically valuable for a number of reasons. In addition to anesthesia, certain NMDA receptor antagonists confer profound analgesia, a highly desirable component of general anesthesia and sedation. Also, NMDA receptor antagonists are neuroprotective under many clinically relevant circumstances (including neuropathic pain states, ischemia, brain trauma, and certain types of convulsions).

There are several NMDA antagonists that are commercially available and have a wide variety of uses. For example, memantine provides rapid and enduring improvement in cognitive, psychological, social and motor impairments of dementia; dextromethorphan is used to relieve coughs; amantadine is an antiviral substance; and ketamine as an anesthetic agent. Certain opioids such as methadone, dextropropoxyphene, and ketobemidone are also classified as NMDA antagonists. MK-801 (dizocilpine maleate) and phencyclidine are not commercially used, and dextromethorphan, which is used commercially are other examples.

There are numerous potential commercial applications for NMDA antagonist formulations without neurotoxicity in supervised medical practice. Indications include, but are not limited to, treatment of dementia, suppression of cough (antitussive), antiviral treatment, treatment of involuntary muscle actions, antidepressant, suppression of addiction, and treatment of withdrawal. For example, ketamine which is useful in accordance with the present invention, can be used as an analgesic for breakthrough pain, anesthesia and sedation. Additional indications for ketamine include traumatic orthopedic injury pain, migraine pain, obstetrical use for end-stage labor pain, central pain, dental pain, and a host of additional conditions associated with acute and chronic, moderate to severe pain.

More specifically, ketamine, an NMDA receptor antagonist, has been in clinical use for over twenty-five years as a dissociative anesthetic and has demonstrated a wide margin of safety when used acutely as an anesthetic agent. Studies demonstrate the analgesic efficacy of ketamine in a variety of diverse indications including patient self-management of pain (U.S. Pat. Nos. 6,248,789 and No. 5,543,434 to Weg), post-operative analgesia (Naguib et al., Can. Anaesth. Soc. J. 1986, 33:16; Dich-Nielsen et al., Acta Anaesthesiol. Scand. 1992, 36:583; Battacharya et al., Ann. Acad. Med. Singapore 1994, 23:456), analgesia in emergency settings for patients suffering from fractures and soft tissue injury (Hirlinger and Pfenninger, Anaesthsist 1987, 36:140), musculoskeletal trauma (Gurnani et al., Anaesth. Intens. Care 1996, 24:32), wound care procedures (Bookwalter, Plastic Surg. Nursing 1994, 14:43; Humphries et al., J. Burn Care Rehabil. 1997, 18:34), management of acute episodes of neuropathic pain attributed to post-herpetic neuralgia (Eide et al., Pain 1994, 58:347), phantom limb pain (Knox et al., Anaesth. Intens. Care 1995, 23:620), nociceptive orofacial pain (Mathisen et al., Pain 1995, 61:215), and cancer pain (Mercadante et al., J. Pain Symptom Manage. 1995, 10:564; Clark and Kalan, J. Pain Symptom. Manage. 1995, 10:310; Fine, J. Pain Symptom Manage. 1999, 17:296; Lauretti et al., Anesthesiology 1999, 90:1528). These studies describe the use of ketamine administered by a variety of routes including transnasal, parenteral, and oral.

Additional anxiolytic agents that may be considered herein, include tryptamine reuptake inhibitors such as buspirone, sertraline, paroxetine, nefazodone and fluxetine; GABA receptor agonists such as benzodiazepines (e.g. diazepam, tofisopam, alprazolam and flutopazepam); corticotropin releasing factor antagonists such as pivagabine; and MAO inhibitors such as amisulpride. However, the preferred agents contemplated and used herein comprise the NMDA receptor antagonists, and particularly, ketamine, and its pharmaceutical salts.

An advantage of the present invention is that there would be no restriction on administration. The composition or dose could be administered by the subject or by a qualified caregiver. Accordingly, the composition and dose may be administered in advance of a minor procedure that would normally not require the availability, attendance or participation of an anesthesiologist, or like specially trained medical caregiver, without arranging for and securing the availability of such professional. The low-dose could be administered in a doctor's office and the patient would experience a rapid recovery.

The present method can be used to treat subjects pre-operatively. For example, the dose of the invention could be administered for systemic distribution and effect in advance of a particular procedure, such as by spraying into the buccal cavity for absorption. Further, the composition and dose of the invention could be administered in a nasal spray, for example, in the amount of 10 mg per spray dose or puff, for 2-5 puffs within one minute intervals, to relieve and overcome e.g. dental phobia. Thus, for example, a patient suffering from severe dental phobia would experience alleviation of the anxiety without any cognitive impairment after self administration of the nasal spray as described. The present method of low-dose premedication could also be used prior to the administration of electroshock therapy to minimize or prevent the onset of pre-treatment apprehension.

Further and with respect to nasal administration, a proprietary sprayer could be used. Likewise, a patch could be provided to serve as a pre-medicant. Intranasal administration could also be conducted by use of a pledgette, wherein the pledgette could be saturated with 10mg/cc to 100mg/cc depending on the capacity of the pledgette, and then placed in the nasal passage. In the instance where the medication is dispensed from a nasal spray, one could use a an applicator designed for closely controlled or metered release, prior to the administration of the therapy in question.

In addition, trans-buccal administration is included. In this instance a dose of from 10mg/cc to 100mg/cc, depending on the capacity of the pledgette, with a range of from 10 mg to 30 mg being exemplary, could be disposed in a pledgette which would be placed in the buccal fold. The pledgette would be retained in the buccal fold for 1 to 2 minutes and removed when the desired anxiolyis is achieved.

The present doses and method of administration is distinguishable from the administration of pediatric doses of an anesthetic, which is known in the art. The distinctions are that the amount or size of the pediatric dose is roughly 10 times that of the present doses, and the goal with pediatric administration is to sedate whereas in the present instance, the goal is to reduce anxiety and avoid sedation. The present invention is intended for adults who can accurately express their feelings and can correspondingly, describe changes in their mood and feelings, so that overmedication and possible dysphoria can be avoided.

A further aspect of the invention is the use of the present method and the general range of doses of the active to promote or achieve a level of relief from anxiety that in the context of the object of the present invention, could be considered a form of conscious sedation. In particular, the method contemplates the self administration of the present composition by the patient, achieving relief from the anxious state with retention of cognitive and emotional stability.

In a further aspect of the invention, the compositions that may be prepared and administered hereby may include other ingredients, such as complementary therapeutic agents, medicaments and the like, for release and treatment of the tissues at the site of injection. The choice and inclusion of such agents may vary within the skill of the art and could be determined by a skilled physician.

### EXAMPLES

The present invention will be better understood from a consideration of the following illustrative examples, wherein all percentages of ingredients are intended to be percent by weight.

### Example I

A unit dose of ketamine was prepared for intranasal administration and comprised a nasal dispenser containing the active in a concentration of 100mg/cc. Administration comprised from 2 to 3 spray discharges of approximately 20-30mg of the active, in an approximate concentration as administered, so that a total of about 0.2-0.3mg/kg. of the active ingredient was dispensed. The unit dose was administered to a 50 year old healthy male who was scheduled for injection therapy of the knee under local anesthesia, 3-5 minutes prior to the local injection of the anesthetic. The unit dose was administered by a conventional nose spray dispenser, in the amount and regime of three spray discharges of the unit dose in alternate nostrils. The subject experienced relief from anxiety and a general calming effect that lasted from 15-30 minutes.

### Example II

A unit dose was in the same manner and amount as in Example I, and was administered to a 48 year old female who suffers from severe dental phobia. The female was scheduled to undergo a dental extraction under local anesthetic. The unit dose was administered in the same manner and frequency as in Example I, prior to the subject travel to the dental office. The subject was sufficiently lucid to be able to drive to the dental office, and an additional dosage was administered upon arrival in the dentist waiting room. The patient underwent the dental procedure without the development of anxiety. In like fashion to the experience report in Example I, the female reported relief from anxiety and a general calming effect that lasted from 15-30 minutes.

### Example III

In this example the same unit dose was prepared and was administered to a subject in accordance with the same regimen as with Examples I and II. In this instance, the subject was a 47 year old female in good health, who required the removal of a foreign object from her hand, under local anesthesia. The subject received the unit dose approximately 5 minutes prior to undergoing the procedure, and reported the same experience and relief as with the subjects in Examples I and II, above.

### Example IV

In this example the same unit dose was prepared and was administered to a subject in accordance with the same regimen as with Examples I-III. In this instance, the subject was a 40 year old female in good health, who underwent the excision of a mole on the neck required, also under local anesthesia. The subject received the unit dose approximately 5 minutes prior to undergoing the procedure, and reported the same experience and relief as with the subjects in Examples I-III, above.

### Example V

An obese 37 year old man 6'3" 300lbs with Psoriatic arthritis involving the neck had a 3CM wood splinter lodged on the sole of his foot. To control anxiety and fear he was treated with three puffs of NIE 2012 (50% ketamine spray, 1/10 ml spray) two minutes apart to treat his anxiety about having his foot examined. 3 minutes later, his foot was examined, the splinter was identified, grasped and removed without incident.

### Example VI

A 62 year old man with a history of NSTEMI heart history and angina that was controlled with cardiac stents presented for dental surgery on the upper mandible area. Two or three puffs of NIE 2012 was administered by the dentist to treat anxiety, fear and apprehension of the required palatine local anesthetic nerve block. Several visits and local anesthetics were required for bone grafting and sinus lift with the same dental phobia and anxiety. In each instance there was calming and manageability for the injection after the NIE 2012 treatment.

### Example VII

A 55 year old female was anxious and phobic about taking a commercial airline flight. The night before the flight she took one puff of NIE 2012 as a test. The next day prior to leaving the departure terminal waiting area to board she took one puff of NIE 2012. She then walked down the gang way and took her seat on the plane without problem. She took another puff a few minutes later when the plan was about to take off.

The method and composition of the invention may be practiced to relieve anxiety that is caused or that develops from the apprehension of surgical procedures, such as eye surgery, eg. lazik surgery, cataract surgery, vitrectomies to treat retinal conditions, and the like. In each instance, the method and composition may be practiced as taught and exemplified herein, by advance administration to the individual in advance of the procedure.

The present method and corresponding composition may be administered in like manner to the computer-managed and controlled system developed and marketed by Ethicon, and known as "SEDASYS." The following is general information regarding the Sedasys System, which is incorporated herein by reference.

The SEDASYS System is a computer-assisted personalized sedation device that delivers the drug propofol for minimal-to-moderate sedation. The device provides comprehensive patient monitoring and limits the depth of sedation by adjusting drug delivery accordingly. The system administers the sedative agent (drug) into the blood stream via intravenous (IV) infusion. The device can detect signs associated with oversedation and can automatically modify or stop infusion.

The four piece system includes:
Bedside Monitoring Unit (BMU) designed to stay with the patient from before the procedure, through the procedure and post-procedure recovery.

Procedure Room Unit (PRU) designed to stay in the procedure room and provides additional patient monitoring. It also contains the sedative (drug) infusion pump controller.

Display monitors and connectors.

Disposable devices for single patient use.

Various publications in addition to the immediately foregoing are cited herein, the disclosures of which are incorporated by reference in their entireties. The citation of any reference herein should not be deemed as an admission that such reference is available as prior art to the instant invention.

While the invention has been described and illustrated herein by references to the specific embodiments, various specific materials, procedures and examples, it is understood that the invention is not restricted to the particular material combinations of material, and procedures selected for that purpose. Indeed, various modifications of the invention in addition to those described herein will become apparent to those skilled in the art from the foregoing description, and such modifications are intended to fall within the scope of the present invention.

The present application and invention further includes the subject matter of the following numbered clauses:
1. A method for inhibiting, ameliorating or treating the development of anxiety in a subject, comprising administering to said subject an NMDA receptor antagonist, in an amount of from about 0.1mg/kg to 0.3mg/kg.
2. The method of clause 1 wherein the NMDA receptor antagonist comprises ketamine, and pharmaceutically acceptable salts thereof.
3. The method of clause 2 wherein said the NMDA receptor antagonist comprises ketamine hydrochloride.
4. The method of clause 1 wherein the NMDA receptor antagonist is administered intranasally.
5. The method of clause 1 wherein the NMDA receptor antagonist is administered transbuccally.
6. The method of clause 1 wherein the NMDA receptor antagonist is administered transdermally.
7. The method of clause 1 wherein the NMDA receptor antagonist is administered intravenously.
8. The method of clause 1, wherein the anxiety is associated with generalized anxiety disorder, obsessive-compulsive disorder, panic disorder, a phobic disorder, acute stress disorder, post-traumatic stress disorder, or mixed anxiety-depression.
9. The method of clause 1 wherein said NMDA receptor antagonist is administered prior to a medical or dental procedure.
10. The method of clause 7 wherein said NMDA receptor antagonist is administered from about 30 minutes to about 2 minutes prior to said medical or dental procedure.
11. The method of clause 1 wherein said NMDA receptor antagonist is administered prior to the administration of electroshock therapy.
12. A pharmaceutical composition which comprises containing about 0.05 mg/kg to about 0.5 mg/kg ketamine hydrochloride, wherein the composition is sub-anesthetic and hypo-analgetic, and is administered as a premedication.
13. The pharmaceutical composition of clause 11, further comprising a suitable carrier selected from the group consisting of water, saline, bicarbonate, sucrose and mixtures thereof.
14. A composition according to clause 11 wherein said composition is prepared in as an aqueous solution.
15. An anxiolytic composition for the prevention, amelioration and/or treatment of anxiety in a subject comprising a sub-anesthetic and hypo-analgetic dosage of an NMDA receptor antagonist.
16. The composition of clause 14, wherein the NMDA receptor antagonist comprises ketamine and its pharmaceutically acceptable salts.
17. The composition of clause 14, wherein the dosage is from about 0.05mg/kg to about 0.5mg/kg.
18. A unit dose of a pharmaceutical composition which comprises an aqueous solution containing about 0.05 mg/kg to about 0.5 mg/kg ketamine hydrochloride, wherein the unit dose is sub-anesthetic and hypo-analgetic, and wherein said dose is administered as a premedication.

## Claims

1. A pharmaceutical composition comprising ketamine hydrochloride for use in inhibiting, ameliorating or treating the development of anxiety in a subject, wherein the composition is administered intranasally in a unit dose amount from about 0.05 mg/kg to about 0.3 mg/kg ketamine hydrochloride.

2. The pharmaceutical composition of claim 1, wherein the unit dose is in an amount from about 0.1 mg/kg to 0.3 mg/kg ketamine hydrochloride.

3. The pharmaceutical composition of claim 1 or claim 2, wherein the unit dose is sub-anesthetic and hypo-analgesic.

4. The pharmaceutical composition of any preceding claim, wherein said composition is administered by the subject as a premedication.

5. The pharmaceutical composition of any preceding claim, wherein the composition is administered from a single-use aerosol or a multi-dose spray device.

6. The pharmaceutical composition of any preceding claim, wherein the anxiety is associated with obsessive-compulsive disorder.

7. The pharmaceutical composition of any one of claims 1-5, wherein the anxiety is associated with generalized anxiety disorder.

8. The pharmaceutical composition of any one of claims 1-5, wherein the anxiety is associated with a panic disorder.

9. The pharmaceutical composition of any one of claims 1-5, wherein the anxiety is associated with a medical procedure.

10. The pharmaceutical composition of any one of claims 1-5, wherein the anxiety is associated with a dental procedure.

11. The pharmaceutical composition of claim 1, wherein the composition further comprises a suitable carrier selected from water, saline, bicarbonate, sucrose and mixtures thereof.
